# EUROPÄISCHE PATENTSCHRIFT

(11) **EP 2 589 349 B1**
(45) Veröffentlichungstag und Bekanntmachung des Hinweises auf die Patenterteilung: **01.04.2015**
(21) Anmeldenummer: 11008747.5
(22) Anmeldetag: 02.11.2011
(51) Int. Cl.: A61B 17/34, A61B 17/00

(54) **Medizinische Vorrichtung zum Einführen von endoskopischen Instrumenten in eine Körperhöhle**
Medical device for inserting endoscopic instruments into a bodily cavity
Dispositif médical pour l'introduction d'instruments endoscopiques dans une ouverture corporelle

(43) Veröffentlichungstag der Anmeldung: 08.05.2013
(73) Patentinhaber: A.M.I. Agency for Medical Innovations GmbH, 6800 Feldkirch (AT)
(72) Erfinder: Egle, Walter, 6842 Koblach (AT)
(74) Vertreter: Hofmann, Ralf U.

(56) Entgegenhaltungen:
- WO-A1-99/16368
- WO-A1-03/000145
- WO-A2-03/011154
- WO-A2-2004/011037

## Beschreibung

Die Erfindung bezieht sich auf eine medizinische Vorrichtung zum Einführen von endoskopischen Instrumenten in eine Körperhöhle eines Patienten bei einer endoskopischen Operation, mit einer Trokarhülse, welche ein distales Ende zur Anordnung in der Körperhöhle aufweist, und mit einem einen Durchgangskanal aufweisenden Anschlussstück, welches im Einsatzzustand der Vorrichtung an einem proximalen Ende der Trokarhülse mit der Trokarhülse verbunden ist, wobei das Anschlussstück (9) einen Zuführabschnitt (10), über welchen sich der Durchgangskanal zum von der Trokarhülse (1) abgelegenen Ende des Anschlussstücks (9) hin trichterförmig erweitert, und einen Einsteckabschnitt (12) aufweist, welcher in das durch Ablängen der Trokarhülse (1) an einer beliebigen Stelle zumindest innerhalb eines Verbindungsabschnitts (32)s der Längserstreckung der Trokarhülse (1) ausbildbare proximale Ende (24) der Trokarhülse (1) einsteckbar ist.

Medizinische Vorrichtungen zum Einführen von chirurgischen Instrumenten in Körperhöhlen sind in unterschiedlichen Ausführungsformen bekannt und werden üblicherweise als Trokare bezeichnet. In älteren und nach wie vor gebräuchlichen Ausführungsformen weisen Trokare eine Metallhülse auf, in welche ein zylindrischer Dorn mit kegeliger Spitze mit geringem Spiel einsteckbar ist, wobei die kegelige Spitze des Dorns aus dem distalen Ende der Metallhülse herausragt. Zur Durchführung einer endoskopischen Operation wird die Metallhülse mit dem eingesteckten Dorn mit ihrem distalen Ende voraus durch äußere Gewebeschichten des Patienten hindurch bis in eine Körperhöhle eingeführt, wobei die kegelige Spitze die äußeren Gewebeschichten durchdringt. Nachdem die kegelige Spitze des Dorns im Inneren der Körperhöhle, beispielsweise Bauchhöhle, angekommen ist, zieht der Chirurg den Dorn aus der Metallhülse heraus. Die Metallhülse bildet nun einen Zugang für endoskopische Instrumente mit der entsprechenden Größe.

Mit der Entwicklung der laparoskopischen Chirurgie, bei welcher die Bauchhöhle mittels Gasüberdruck aufgeblasen wird, um damit Raum für den chirurgischen Eingriff innerhalb der Bauchhöhle zu schaffen, haben Trokare mit Ventilschleusen Eingang in die chirurgische Praxis gefunden. Diese Ventilschleusen der Trokare verhindern einen Gasverlust aus der Bauchhöhle, wenn der Trokar eingesetzt ist sowie beim Einführen und beim Bedienen von endoskopischen Instrumenten durch den Trokar. Am Ende der Operation zieht der Chirurg die zuvor gesetzten Trokare aus der entsprechenden Körperhöhle und verschließt die Trokareinstiche mit einer chirurgischen Naht.

Ein chirurgischer Trokar mit einer längsverstellbaren Hülse ist beispielsweise aus der US 5,746,720 bekannt. Damit kann die Metallhülse an die unterschiedlichen Wanddicken, die am Patienten zu überwinden sind, angepasst werden. In US 5,697,913 wird ein Trokar gezeigt, dessen Hülse mit radialen Rillen versehen ist. Damit soll nach dem Setzen der Trokarhülse, z.B. in der Bauchdecke eines Patienten, ein Verrutschen derselben verhindert werden. Um in einer Trokarhülse mit großem Innendurchmesser auch Instrumente mit einem kleinen Außendurchmesser ohne Gasverlust aus der aufgeblasenen Körperhöhle durchführen zu können, werden verschiedene Ventilschleusensysteme eingesetzt. Eine derartige Vorrichtung wird in der US 5,104,383 gezeigt. In US 5,391,156 wird eine flexible Trokarhülse zum Einsatz zwischen den Rippen gezeigt, wenn Zugang zur Thoraxhöhle benötigt wird. In diesem Fall kann auf den Einsatz einer Ventilschleuse am Trokar verzichtet werden, da bei diesen Eingriffen ohne Gasüberdruck in der Thoraxhöhle gearbeitet wird. US 5,792,113 zeigt einen Trokar mit einer Ventilschleuse, welche den Einsatz von dünnen Instrumenten durch einen Trokar größeren Durchmessers erlaubt, ohne dass zusätzliche Reduzierventilklappen eingesetzt werden müssen.

Diese vorbekannten Trokare sind nicht zum Einsatz mit endoskopischen Instrumenten ausgebildet, die nur einen Außendurchmesser von 4mm oder weniger aufweisen, sodass sie nur bedingt oder gar nicht für den Einsatz in modernen chirurgischen Verfahren geeignet sind. Auch ist üblicherweise beim Durchführen von chirurgischen Instrumenten durch die Ventilschleuse dieser bekannten Systeme ein hoher Reibungswiderstand zu überwinden, welcher in der Folge das Manipulieren des durch die Trokarhülse durchgeführten chirurgischen Instruments behindert.

Bei modernen endoskopischen chirurgischen Verfahren, die auch als Laparoskopie, Thoraskopie, SILS (Single Incision Laparoscopic Searchery) oder als NOTES (Natural Orifice Transluminal Endoscopic Surgery) bezeichnet werden, müssen durch einen möglichst kleinen Trokar mit einem Innendurchmesser von 5mm oder weniger, chirurgische Instrumente sicher und kontaminationsfrei, teilweise mehrfach, in das Operationsgebiet eingebracht werden. Ziel dieser modernen chirurgischen Verfahren ist eine weitere Reduktion des Operationstraumas und ein verbessertes kosmetisches Ergebnis (narbenloses Operieren) durch die Verwendung von chirurgischen Instrumenten mit Schaftdurchmessern von 4mm oder weniger, häufig im Bereich von 2mm bis 3mm.

Eine Vorrichtung zum Einführen von endoskopischen Instrumenten in eine Körperhöhle eines Patienten bei einer endoskopischen Operation, welche eine Trokarhülse und ein mit dieser verbundenes Anschlussstück umfasst, geht aus der EP 0956060 B1 hervor. Die kollabierbare Trokarhülse besitzt hier anschließend an ihr proximales Ende einen sich zur ihrem proximalen Ende hin konisch erweiternden Abschnitt. Das proximale Ende der Trokarhülse ist hierbei an einem Griff zur Handhabung der Vorrichtung angeschlossen. Je nach Dicke der Gewebeschichten zwischen dem Außenraum und der Körperhöhle steht die Trokarhülse mehr oder weniger weit innen und außen aus den Körperschichten heraus. Durch diese an sich überflüssige Länge der Trokarhülse wird die Handhabung der chirurgischen Instrumente erschwert. Bei einer zunehmenden Flexibilität dieser Trokarhülse würde auch die Handhabung beim Einsetzen und Herausziehen von chirurgischen Instrumenten zunehmend erschwert. Diese Trokarhülse muss daher relativ formstabil ausgebildet sein, sodass eine Gasdichtheit allein durch das Kollabieren der Trokarhülse nicht möglich ist.

Aus der WO 02/051323 A1 geht eine ausdehnbare Trokarhülse hervor, in welche ein starres Röhrchen eingeführt wird, welches als Dilatationstrokar bezeichnet wird und durch das hindurch die chirurgischen Instrumente eingeführt werden.

Aus der US 5,176,649 A geht eine teilweise kollabierbare Trokarhülse hervor. Eine Gasdichtheit wird hier nicht erreicht und eine komfortable Handhabung, insbesondere beim Einsetzen und Herausziehen von chirurgischen Instrumenten ist nicht gewährleistet.

Aus der US 2007/0213675 A1 geht ein Trokar mit einer starren Trokarhülse hervor, welche durch innerhalb und außerhalb der von der Trokarhülse durchsetzten Gewebeschichten liegenden distalen und proximalen Ringballone in ihrer Lage fixiert ist.

Eine Vorrichtung der eingangs genannten Art geht aus der WO 03/011154 A2 hervor, welche die Merkmale des Oberbegriffs von Anspruch 1 offenbart. An einem Anschlussstück, welches eine Dichtung aufweist, ist eine Hülse ange-bracht, die radial expandierbar ist. Durch das Anschlussstück und die Hülse ist ein medizinisches Instrument einführbar, beispielsweise ein Trokar mit einer Dichtung.

Die WO 03/000145 A1 beschreibt ein Trokar mit einem trichterförmigen Anschlussstück, in welches eine elastische Trokarhülse eingesteckt ist. Zur Abdichtung der Trokarhülse dient eine Umschließungseinrichtung mit einem elastischen Kunststoffkörper und darin angeordneten Druckelementen.

Aus der WO 99/16368 A1 geht die Verwendung eines mit einem Füllmedium befüllbaren Ringballons zur Verankerung einer starr ausgebildeten Trokarhülse gegen ein Herausziehen aus einer Körperhöhle hervor.

Aufgabe der Erfindung ist es eine Vorrichtung der eingangs genannten Art bereitzustellen, die bei einer einfachen Ausbildung eine vorteilhafte Handhabung ermöglicht. Erfindungsgemäß gelingt dies durch eine Vorrichtung mit den Merkmalen des Anspruchs 1.

Bei der Vorrichtung gemäß der Erfindung weist das mit der Trokarhülse zu verbindende Anschlussstück einen Zuführabschnitt, über welchen sich der Durchgangskanal durch das Anschlussstück zum proximalen Ende des Anschlussstücks hin trichterförmig erweitert, und einen Einsteckabschnitt auf, der das distale Ende des Anschlussstücks bildet. Der Einsteckabschnitt ist zur Verbindung des Anschlussstücks mit der Trokarhülse in ein proximales Ende der Trokarhülse einsteckbar, wobei dieses proximale Ende der Trokarhülse durch Ablängen der Trokarhülse an einer beliebigen Stelle zumindest innerhalb eines Abschnitts der Längserstreckung der Trokarhülse ausgebildet werden kann. Dieser Abschnitt der Längserstreckung der Trokarhülse, über den nach Ablängen der Trokarhülse der Einsteckabschnitt des Anschlussstücks in die Trokarhülse eingesteckt und mit dieser verbunden werden kann, wird hier als Verbindungsabschnitt der Trokarhülse bezeichnet. Wenn die Trokarhülse über ihre gesamte Längserstreckung gleich ausgebildet ist, so könnte die gesamte Längserstreckung der Trokarhülse als Verbindungsabschnitt angesehen werden. Alternativ könnte dann derjenige Abschnitt der Längserstreckung der Trokarhülse als Verbindungsabschnitt angesehen werden, der maximal entfernt wird, wenn die Trokarhülse bei der niedrigsten Dicke der zu durchsetzenden Gewebeschichten, für welche die Trokarhülse vorgesehen ist, eingesetzt wird.

Nach dem Einsetzen der Trokarhülse durch die Haut und die daran anschließenden Gewebeschichten des Patienten, wobei die Trokarhülse bis in eine Körperhöhle des Patienten ragt, kann die Trokarhülse somit je nach der Dicke der durchsetzten Gewebeschichten an einer geeigneten Stelle innerhalb des Verbindungsabschnitts abgelängt werden, wonach der Einsteckabschnitt in das so gebildete proximale Ende der Trokarhülse eingesteckt werden kann. Dadurch kann die Vorrichtung für ihren Einsatzzustand (= Verwendungszustand), in welchem eine endoskopische Operation mit einem die Vorrichtung durchsetzenden Instrument durchgeführt wird, an die jeweiligen Gegebenheiten angepasst werden. Durch das Vermeiden einer unnötigen Länge des Trokars wird die Handhabung der endoskopischen Instrumente vereinfacht, insbesondere kann ein überstehender, abknickender Abschnitt vermieden werden, welcher das Einführen eines Instruments deutlich erschweren würde.

Vorteilhafterweise kann die Trokarhülse an der gewünschten Stelle des Verbindungsabschnitts mit einer manuell bedienten OP-Schere, wie sie für das Durchtrennen von weichem Körpergewebe eingesetzt wird, durchgeschnitten werden.

Die Trokarhülse ist günstigerweise zumindest über den Abschnitt ihrer Längserstreckung, über welchen sie kollabierbar ausgebildet ist, bereits durch den Gewebedruck der Gewebeschichten des Patienten, durch welche sie durchgeführt ist, vollständig kollabierbar. Vollständig kollabierbar bedeutet, dass der innere Hohlraum nach dem Kollabieren geschlossen ist, sich also der Öffnungsquerschnitt der Trokarhülse auf 0 verringert hat. Es kann dadurch eine Gasdichtheit der Trokarhülse erreicht werden, um den Gasüberdruck in der aufgeblasenen Körperhöhle, beispielsweise Bauchhöhle, zumindest im Wesentlichen zu halten. Beim Einführen eines chirurgischen Instruments durch die Trokarhülse, wird dann die flexible Wand der Trokarhülse gegen den Schaft des Instruments gedrückt, wobei ebenfalls eine Gasdichtheit erreicht werden kann.

Günstigerweise ist hierzu die Trokarhülse derart ausgebildet, dass zumindest über den kollabierbaren Abschnitt Wandabschnitte der Trokarhülse, die bezogen auf den Querschnitt der Trokarhülse gegenüberliegen, durch eine Kraft bis auf Anlage zusammendrückbar sind, welche weniger als 6N beträgt, vorzugsweise weniger als 3N. Diese Kraft wirkt hierbei an einer Stelle von außen auf einen Wandabschnitt der Trokarhülse ein, wobei die Kraft rechtwinkelig zur Längserstreckung der Trokarhülse und parallel zur Oberflächennormalen des Wandabschnitts steht, während der gegenüberliegende Wandabschnitt gegen eine Verschiebung in Richtung der einwirkenden Kraft abgestützt ist. Um den Einfluss der Schwerkraft auszuschalten, wird hierbei die Trokarhülse mit ihrer Längserstreckung vertikal ausgerichtet.

Erfindungsgemäß ist die Trokarhülse zumindest über den Abschnitt ihrer Längserstreckung, über welchen sie kollabierbar ausgebildet ist, ohne Formstabilität bzw. mit einer so geringen Formstabilität ausgebildet, dass sie bereits unter dem Einfluss der Schwerkraft kollabiert. Hierbei faltet sich die auf einer Unterlage mit horizontaler Auflagefläche abgelegte Trokarhülse, deren Längserstreckung somit horizontal ausgerichtet ist, zumindest über den Abschnitt ihrer Längserstreckung, über welchen sie kollabierbar ausgebildet ist, zusammen, wobei bezogen auf den Querschnitt gegenüberliegende Wandabschnitte der Trokarhülse aufeinander aufliegen.

Die erfindungsgemäße Vorrichtung kann somit in vorteilhaften Ausführungsformen ohne zusätzliche Ventilvorrichtungen ausgebildet sein. Zusätzliche Ventilvorrichtungen, beispielsweise um die Gasdichtheit weiter zu verbessern, können in anderen Ausführungsformen aber vorgesehen sein.

Die Länge des Verbindungsabschnitts der Trokarhülse, über welchen das Anschlussstück durch Einstecken eines Einsteckabschnitts mit der Trokarhülse verbunden werden kann, nachdem die Trokarhülse an der entsprechenden Stelle des Verbindungsabschnitts abgeschnitten worden ist, beträgt vorteilhafterweise mindestens 50mm, vorzugsweise mindestens 60mm. In einer bevorzugten Ausführungsform ist die Trokarhülse über ihre gesamte Länge gleich ausgebildet, sodass die Trokarhülse grundsätzlich an einer beliebigen Stelle abgeschnitten werden kann und der Einsteckabschnitt des Anschlussstücks in das abgeschnittene Ende eingesteckt werden kann.

Im bezogen auf ihren Querschnitt zu einem Kreisring geöffneten Zustand, aber im entspannten Zustand des Materials der Trokarhülse, also im ungedehnten Zustand der Trokarhülse, weist die Trokarhülse vorteilhafterweise zumindest über den Verbindungsabschnitt einen konstanten Innendurchmesser auf.

Die Länge der Trokarhülse, beispielsweise zum Einsatz in einer laparoskopischen Operation, beträgt im Auslieferungszustand vorzugweise mindestens 120mm, besonders bevorzugt mindestens 150mm.

Die Trokarhülse weist vorteilhafterweise ein textiles Material auf, insbesondere ein ihren inneren Hohlraum umgebendes Gewebe oder eine ihren inneren Hohlraum umgebende Maschenware, insbesondere ein Gewirke, wobei die Trokarhülse vorzugweise insgesamt aus einem solchen textilen Material besteht, also keine zusätzlichen nichttextilen Lagen aufweist.

Um die Vorrichtung gegen ein Herausziehen der Trokarhülse aus der Körperhöhle während der Operation zu verankern, ist erfindungsgemäß ein fest mit der Trokarhülse verbundener, mit einem Füllmedium befüllbarer distaler Ringballon vorgesehen. Dieser liegt während der Operation innerhalb der Körperhöhle, wobei er sich an der die Körperhöhle begrenzenden Gewebeschicht abstützen kann. Der distale Ringballon weist vorzugsweise einen Abstand vom distalen Ende der Trokarhülse auf, der kleiner als 5mm ist. Besonders bevorzugt schließt er direkt an das distale Ende der Trokarhülse an.

Um die Vorrichtung während der Operation gegen ein weiteres Hineinschieben in die Körperhöhle hinein zu verankern, ist in einer vorteilhaften Ausführungsform weiters ein Verankerungsteil vorgesehen. In einem geöffneten Zustand ist dieses gegenüber der Trokarhülse in deren Längsrichtung verschiebbar und/oder an verschiedenen Stellen der Längserstreckung der Trokarhülse an dieser anlegbar. In einem Klemmzustand ist das Verankerungsteil gegen eine Verschiebung gegenüber der Trokarhülse gesichert und kann sich gegen die Haut (oder eine darauf aufgelegte Lage) abstützen. Als Verankerungsteil kann vorteilhafterweise ein proximaler Ringballon vorgesehen sein. Dieser ist, zumindest vor dem (vollständigen) Befüllen mit dem Befüllmedium, an eine gewünschte Stelle der Längserstreckung der Trokarhülse verschiebbar. Nachdem Befüllen ist er mit der Trokarhülse verklemmt.

Weitere Vorteile und Einzelheiten der Erfindung werden im Folgenden anhand der beiliegenden Zeichnung erläutert. In dieser zeigen:
Fig. 1 eine schematische Seitenansicht der Vorrichtung, im auseinandergenommenen Zustand der Teile;
Fig. 2 eine schematische Darstellung nach dem Durchführen durch die Gewebeschichten des Patienten und vor dem Befüllen des distalen Ringballons;
Fig. 3 eine Darstellung entsprechend Fig. 2 nach dem Befüllen des distalen Ringballons;
Fig. 4 eine Darstellung entsprechend Fig. 3 nach dem Herausziehen des Dorns;
Fig. 5 eine Darstellung entsprechend Fig. 4 nach dem Aufsetzen und Befüllen des proximalen Ringballons und vor dem Ablängen der Trokarhülse;
Fig. 6 eine Darstellung entsprechend Fig. 5 nach dem Ablängen der Trokarhülse und der Verbindung mit dem Anschlussstück, also im Einsatzzustand der Vorrichtung;
Fig. 7 eine Darstellung entsprechend Fig. 6 mit einem durch die Vorrichtung durchgesteckten endoskopischen Instrument;
Fig. 8 einen Querschnitt entlang der Linie AA von Fig. 1;
Fig. 9 einen Längsmittelschnitt durch das Anschlussstück;
Fig. 10 einen Längsmittelschnitt durch den distalen Endabschnitt der Trokarhülse mit dem daran angebrachten Ringballon (die Trokarhülse am distalen Ende etwas kürzer als in den Figuren zuvor dargestellt);
Fig. 11 einen Längsmittelschnitt durch den proximalen Ringballon und dessen Anschlussröhrchen (in einer gegenüber den Figuren zuvor geringfügig modifizierten Ausführungsform);
Fig. 12 eine schematische Darstellung eines Querschnitts durch die Trokarhülse im durch eine an gegenüberliegenden Umfangsstellen der Trokarhülse punktuell einwirkenden Kraft kollabierten Zustand;
Fig. 13 eine schematische Darstellung eines Querschnitts durch eine durch die Schwerkraft kollabierte Trokarhülse;
Fig. 14 und Fig. 15 schematische Darstellungen von weiteren möglichen Ausführungsformen.

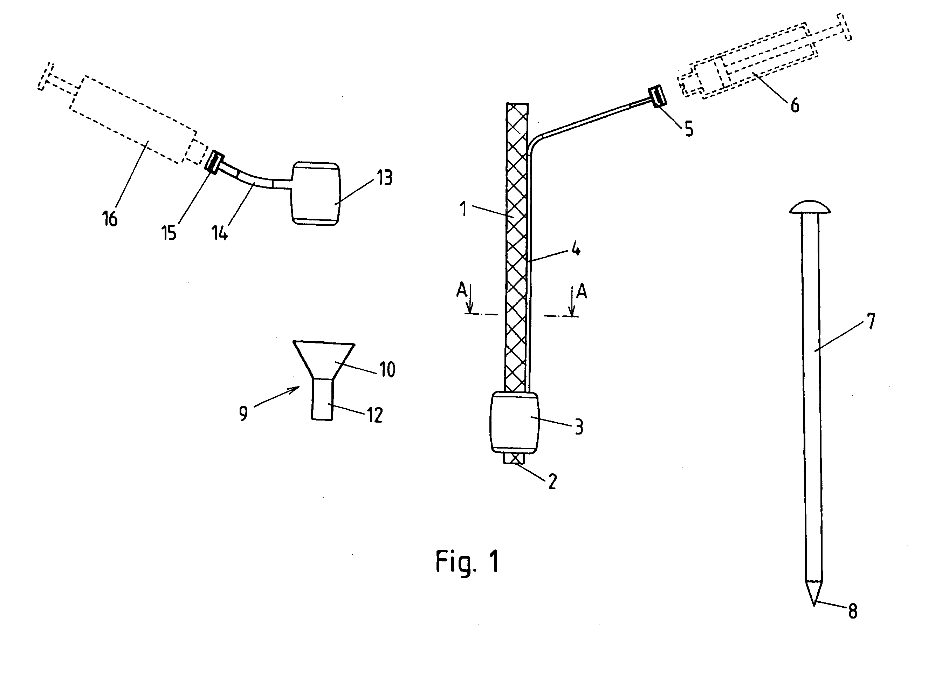

Eine Ausführungsform einer erfindungsgemäßen Vorrichtung im auseinandergenommenen Zustand ihrer Teile ist in Fig. 1 dargestellt. Die Vorrichtung umfasst eine zumindest über einen Abschnitt ihrer Längserstreckung flexible, im Querschnitt kollabierbare Trokarhülse 1, deren distales Ende 2 zur Anordnung in einer Körperhöhle eines Patienten vorgesehen ist. Vorzugsweise ist die Trokarhülse über ihre gesamte Länge mit gleichen Eigenschaften ausgebildet, insbesondere mit gleichem Innen- und Außendurchmesser, gleicher Flexibilität und gleicher Elastizität. Im distalen Endbereich der Trokarhülse 1 ist mit der Trokarhülse 1 ein distaler Ringballon 3 verbunden, der sich um den äußeren Umfang der Trokarhülse 1 herum erstreckt, beispielsweise an diesem angeklebt ist. Zum Befüllen des distalen Ringballons 3 mit einem Befüllmedium, welches beispielsweise von Luft, sterilem Wasser oder einer sterilen Kochsalzlösung gebildet wird, dient ein Befüllröhrchen 4 (=Füllkatheter). Dieses verläuft in der Längsrichtung der Trokarhülse von einem proximalen Bereich der Trokarhülse 1 bis zum distalen Ringballon 3 und ist hierbei mit der Außenseite der Trokarhülse 1 verbunden. Am anderen Ende des Befüllröhrchens 4 ist ein Anschluss 5 mit integriertem Rückschlagventil angeordnet, an den eine handelsübliche Spritze 6 angeschlossen werden kann, die in Fig. 1 mit strichlierten Linien angedeutet ist.

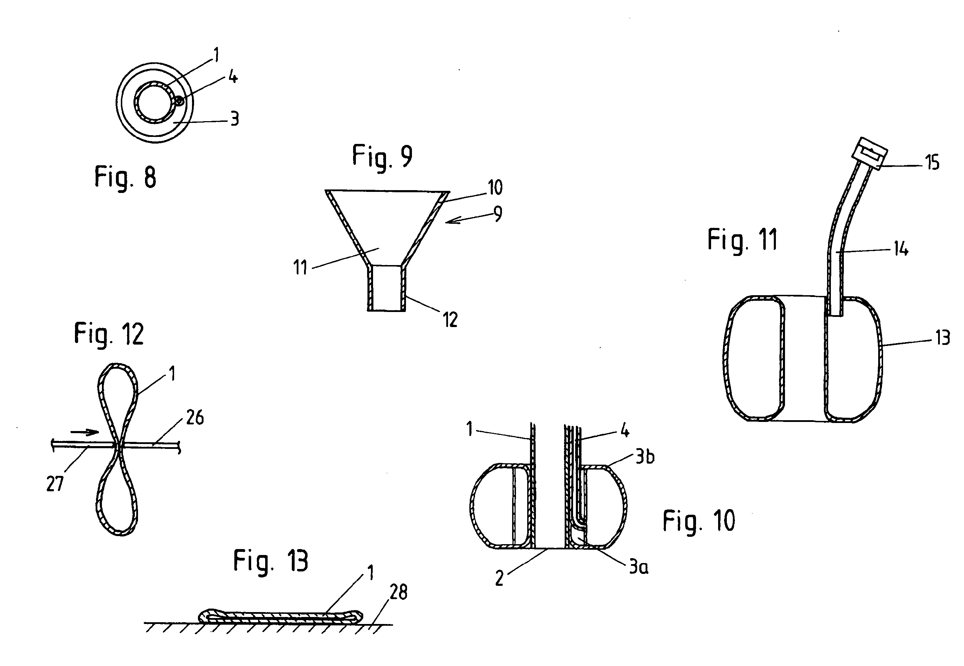

Fig. 10 zeigt beispielhaft eine mögliche Ausbildungsform des distalen Ringballons 3 im Längsschnitt. Ein Basisabschnitt 3a des Ringballons ist mit der äußeren Oberfläche der Trokarhülse 1 verklebt und an diesem Basisabschnitt 3a ist eine dehnbare Membran 3b mit ihren beiden Enden befestigt. Das Befüllröhrchen 4 setzt sich durch den Basisabschnitt 3a fort und mündet in den vom Basisabschnitt 3a und der Membran 3b umgebenen Hohlraum.

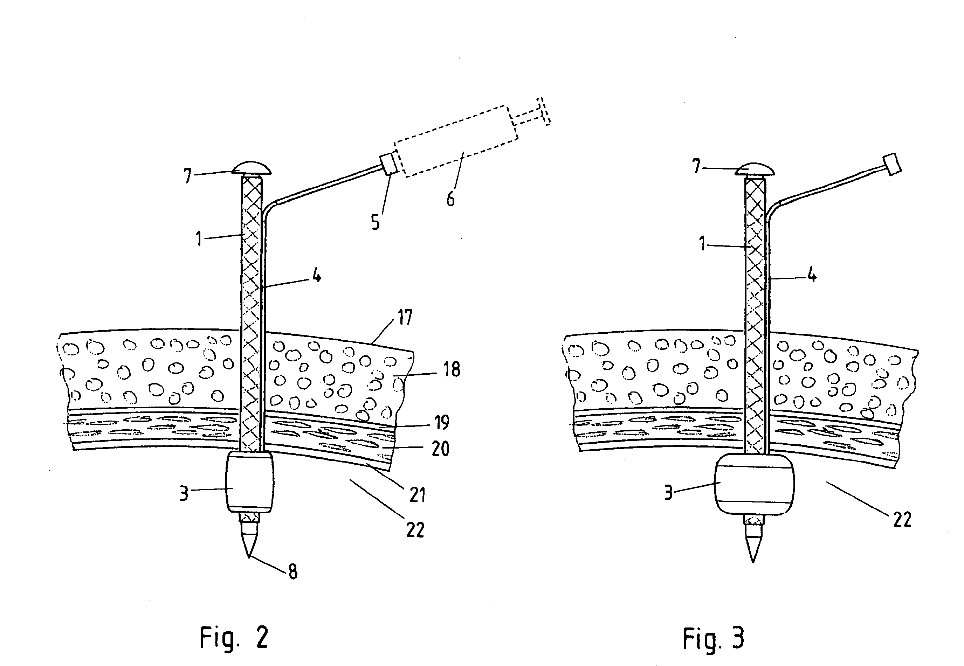

In Fig. 10 ist das distale Ende der Trokarhülse 1 bündig zum distalen Rand des Ringballons 3 dargestellt, wie dies bevorzugt ist. In Fig. 1 (ebenso wie in den Fig. 2 bis 7 und 14 ist das distale Ende 2 der Trokarhülse 1 der Übersichtlichkeit halber aber etwas über den distalen Rand des Ringballons 3 hinaus verlaufend dargestellt).

Zum Einsetzen der Trokarhülse 1 mit ihrem distalen Ende 2 voraus bis in die Körperhöhle eines Patienten dient ein Dorn 7, der von proximal in den inneren Hohlraum der Trokarhülse 1 eingeschoben wird. Im vollständig eingeschobenen Zustand ragt die Spitze (=das kegelige Ende) 8 des Dorns 7 aus dem distalen Ende 2 der Trokarhülse 1 heraus.

Im Auslieferungszustand ist der Dorn 7 günstigerweise bereits in die Trokarhülse 1 eingesetzt. Dies muss dann nicht mehr vom Operateur veranlasst werden.

Die Vorrichtung umfasst weiters ein Anschlussstück 9. Dieses besitzt einen Zuführabschnitt 10, über welchen sich der das Anschlussstück 9 durchsetzende Durchgangskanal 11 (vgl. Fig. 9) zum proximalen Ende des Anschlussstücks 9 (=freies Ende des Zuführabschnitts 10) hin trichterförmig erweitert. Weiters besitzt das Anschlussstück 9 einen rohrstutzenartigen Einsteckabschnitt 12 zur Verbindung mit der Trokarhülse 1, wie dies weiter unten genauer erläutert wird.

Die Vorrichtung umfasst weiters einen proximalen Ringballon 13. Dieser kann von proximal her auf die Trokarhülse 1 (und das entlang von dieser an deren Außenseite geführte Befüllröhrchen 4) an die gewünschte Stelle der Trokarhülse 1 aufgeschoben werden, wie dies ebenfalls weiter unten noch genauer erläutert wird. Über ein Befüllröhrchen 14 und einen Anschluss 15 mit integriertem Rückschlagventil kann der proximale Ringballon mittels einer handelsüblichen Spritze 16, die in Fig. 1 durch strichlierte Linien angedeutet ist, mit einem Befüllmedium befüllt werden, beispielsweise mit Luft oder Wasser. In Fig. 1 mündet das Befüllröhrchen 14 radial in den proximalen Ringballon 13. Auch eine axiale Einmündung ist beispielsweise möglich, wie dies in Fig. 11 dargestellt ist.

Fig. 2 zeigt den Zustand nach dem Einbringen der Trokarhülse durch die Haut 17 und die weiteren Gewebeschichtn 18 - 21 (Fettgewebe, Faszie-Muskel, Muskel und Faszie-Bauchfell) bis in die in diesem Ausführungsbeispiel von der Bauchhöhle gebildete Körperhöhle 22. Die Trokarhülse 1 mit dem darin eingesetzten Dorn 7 wird soweit durch die Gewebeschichtn 17 - 21 durchgeführt und in die Körperhöhle 22 eingeführt, bis der distale Ringballon 3 innerhalb der Körperhöhle 22 liegt.

In der Folge wird der distale Ringballon 3 mit dem Befüllmedium befüllt. Dieser Zustand ist in Fig. 3 dargestellt. Durch das Befüllen des distalen Ringballons 3 mit dem Befüllmedium vergrößert sich der äußere Durchmesser des Ringballons 3. Wenn der distale Ringballon 3 an der die Körperhöhle 22 begrenzenden Gewebeschicht 21 anliegt, ist die Trokarhülse 1 somit gegen ein weiteres Herausziehen aus der Körperhöhle 22 gesichert.

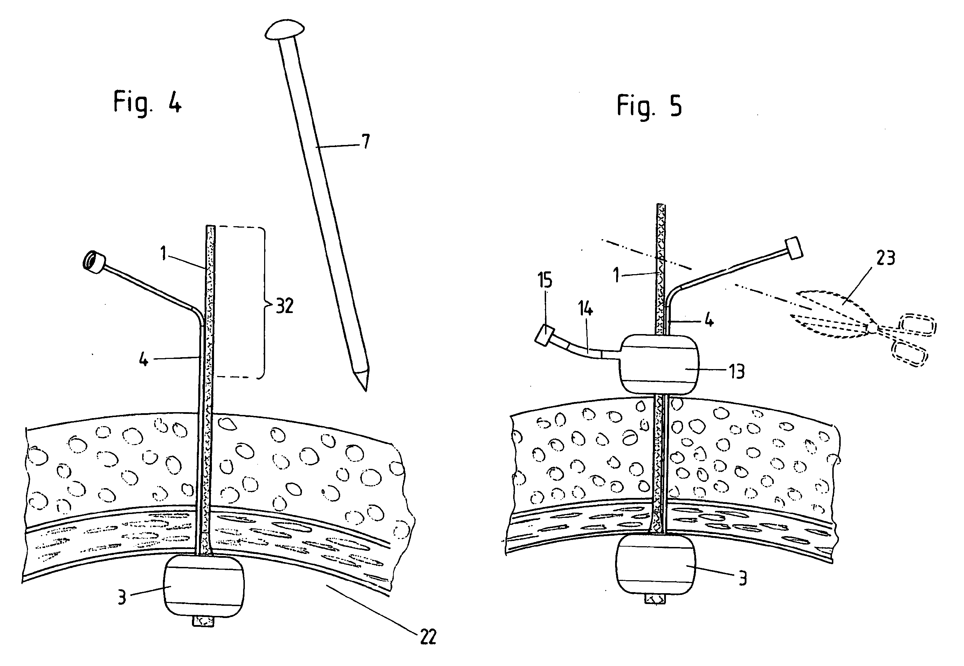

Der Dorn 7 wird in der Folge aus der Trokarhülse 1 herausgezogen, vgl. Fig. 4. Zumindest im von den Gewebeschichtn 17 - 21 umgebenen Bereich kollabiert daraufhin die Trokarhülse 1 durch den vom umgebenden Gewebe ausgeübten Druck so, dass sich der innere Hohlraum der Trokarhülse 1 verschließt. Wenn die Trokarhülse 1 ohne Formstabilität ausgebildet ist, so können sich auch im außerhalb der Gewebeschichtn 17 - 21 gegenüberliegende Wandabschnitte der Trokarhülse 1 aneinander anlegen. Weiters würde dann die Trokarhülse 1 durch die einwirkende Schwerkraft, soweit sie nicht durch das Befüllröhrchen 4 gestützt ist, umknicken (nicht dargestellt in Fig. 4).

Der distale Endabschnitt der Trokarhülse 1 wird vorteilhafterweise durch eine Verbindung mit dem formstabil ausgebildeten Basisabschnitt 3a des distalen Ringballons 3 offen gehalten. Das Zurückziehen eines durch die Trokarhülse 1 in die Körperhöhle 22 eingeführten endoskopischen Instruments wird dadurch erleichtert. Der distale Endabschnitt der Trokarhülse 1 könnte auch von einem formstabilen Röhrchen gebildet werden, an welches der flexible Abschnitt der Trokarhülse 1 angeschlossen ist. Der distale Ringballon könnte dann auf diesem formstabilen Abschnitt der Trokarhülse 1 angeordnet sein.

In der Folge wird der proximale Ringballon 13 auf die Trokarhülse 1 und das außen entlang von dieser geführte Befüllröhrchen 4 aufgeschoben, bis er an der Haut 17 anliegt, wobei durch Zug an der Trokarhülse 1 sichergestellt wird, dass der distale Ringballon 3 an der die Körperhöhle 22 begrenzenden Gewebeschicht 21 anliegt. In der Folge wird der proximale Ringballon 13 mit dem Befüllmedium befüllt. Hierbei verringert sich sein innerer Durchmesser, sodass er auf der Trokarhülse 1 und dem entlang von dieser geführten Befüllröhrchen 4 festgeklemmt wird. Auch der äußere Durchmesser des proximalen Ringballons 13 kann sich gegebenenfalls vergrößern.

Somit ist die Trokarhülse 1 gegen eine Verschiebung in ihre Längsrichtung gesichert.

Durch den proximalen Ringballon 13 kann auch eine (zusätzliche) Abdichtung gegen einen Austritt von Gas aus der Körperhöhle 22 erreicht werden.

In der Folge wird ein je nach der Dicke der durchsetzten Gewebeschichtn 17 - 21 unterschiedlich langer proximaler Abschnitt der Trokarhülse 1 entfernt, d. h. die Trokarhülse 1 wird an einer geeigneten Stelle abgelängt. Eine hierzu verwendbare Schere 23 ist in Fig. 5 durch strichlierte Linien angedeutet. Zuvor wird, soweit erforderlich, das Befüllröhrchen 4 über den zu entfernenden Abschnitt der Trokarhülse 1 von dieser getrennt. Das Befüllröhrchen 4 ist hierzu günstigerweise so mit der Trokarhülse 1 verbunden, dass dieses durch Zug von der Trokarhülse 1 abgezogen werden kann.

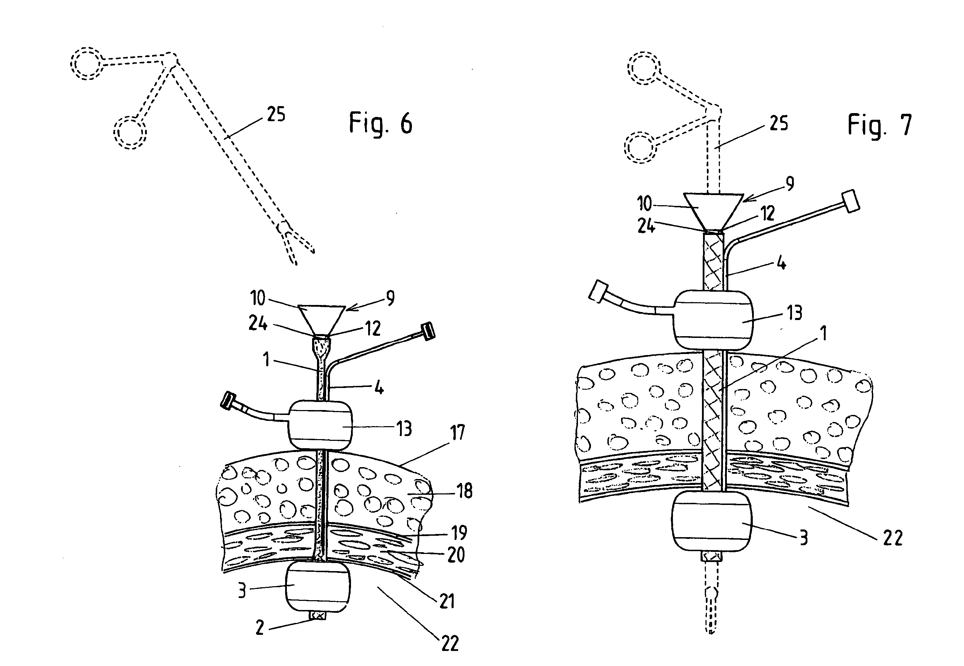

In das an der gewünschten Stelle ausgebildete proximale Ende 24 der Trokarhülse 1 wird der Einsteckabschnitt 12 des Anschlussstücks 9 eingesetzt, vgl. Fig. 6. Die Trokarhülse 1 besitzt hierzu vorteilhafterweise eine entsprechende Elastizität, sodass sie beim Einstecken des Einsteckabschnitts 12 gedehnt wird und durch ihre Elastizität auf dem Einsteckabschnitt 12 gehalten wird. Dieser Zustand ist in Fig. 6 dargestellt.

Damit ist der Einsatzzustand der Vorrichtung hergestellt, in welchem die Vorrichtung einen Trokar ausbildet, durch den hindurch eine endoskopische Operation durchgeführt werden kann.

Hierbei kann ein beispielhaft in den Fig. 6 und 7 durch strichlierte Linien angedeutetes endoskopisches Instrument 25 durch die Trokarhülse 1 in die Körperhöhle 22 eingeführt werden, um in der Folge in der Körperhöhle 22 mit dem endoskopischen Instrument 25 Operationsschritte durchzuführen. Der durch die Trokarhülse 1 durchgeführte Zustand eines endoskopischen Instruments 25 ist in Fig. 7 dargestellt. Das endoskopische Instrument 25 ist als Schaftinstrument ausgebildet, d. h. besitzt einen längserstreckten Schaft, an dessen distalen Ende ein Funktionselement (beispielsweise ein Greifer, ein Schneidelement, usw.) angeordnet ist und an dessen proximalen Ende eine Handhabe angeordnet ist.

Der Außendurchmesser des Schafts des Schaftinstruments und des Funktionselements (im geschlossenen Zustand) des Schaftinstruments beträgt vorteilhafterweise weniger als 4mm.

Der Innendurchmesser der Trokarhülse 1 beträgt vorteilhafterweise im (bezogen auf ihren Querschnitt) zu einem Kreisring geöffneten Zustand der Trokarhülse 1, aber im entspannten Zustand des Materials der Trokarhülse 1 weniger als 7mm, besonders bevorzugt höchsten 5mm.

Je nach insgesamter Dicke der durchsetzten Gewebeschichten 17 - 21 wird günstigerweise ein mehr oder weniger großer proximaler Teil der Trokarhülse 1 entfernt und in das so ausgebildete proximale Ende 24 der Trokarhülse 1 der Einsteckabschnitt 12 des Anschlussstücks 9 eingesteckt. Die maximal entfernbare Länge der Trokarhülse 1 ergibt sich aus der insgesamten Dicke der Gewebeschichten 17- 21 und den benötigten Überständen der Trokarhülse 1 innen und außen. Die maximal entfernbare Länge für die geringste insgesamte Dicke der Gewebeschichten 17 - 21, für deren Einsatz die Trokarhülse 1 vorgesehen ist, kann dann als Verbindungsabschnitt 32 bezeichnet werden, der beispielhaft in Fig. 4 eingezeichnet ist. Je nach Patienten kann die Trokarhülse 1 intraoperativ an einer gewünschten Stelle des Verbindungsabschnitts 32 abgeschnitten werden.

Nach der Operation wird der distale Ringballon 3 entleert und die Vorrichtung aus dem Patienten herausgezogen, worauf der Einstich mit einer chirurgischen Nadel geschlossen wird.

Die Trokarhülse 1 ist jedenfalls über einen Abschnitt ihrer Längserstreckung (welcher im eingesetzten Zustand von Gewebeschichtn 17 - 21 umgeben wird) unter einem relativ geringen Druck derart kollabierbar ausgebildet, dass gegenüberliegende Wandabschnitte zur gegenseitigen Anlage gelangen. Fig. 12 zeigt eine Anordnung zur Bestimmung der benötigten, punktuell auf die Trokarhülse 1 ausgeübten Kraft, um gegenüberliegende Wandabschnitte zur Anlage zu bringen. Die vertikal ausgerichtete Trokarhülse 1 ist auf einer Seite von einem stiftförmiges Abstützteil 26 abgestützt und von der gegenüberliegenden Seite her wird ein stiftförmiges Zustellteil 27 gegen die Trokarhülse 1 mit einer definierten Kraft verfahren, bis die gegenüberliegenden Wandabschnitte, an denen das Abstützteil und das Zustellteil 26, 27 anliegen, in gegenseitigen Kontakt gebracht sind. Die hierfür benötigte Kraft beträgt jedenfalls weniger als 6N, vorzugsweise weniger als 3N. Um eine im Wesentlichen punktuelle Bestimmung der Kraft durchzuführen, betragen die Außendurchmesser des Abstützteils 26 und des Zustellteils 27 weniger als 1/5 des Außendurchmessers der Trokarhülse 1.

Die Trokarhülse 1 ist erfindungsgemäß zumindest über einen Abschnitt ihrer Längserstreckung (der im eingesetzten Zustand von den Gewebeschichtn 17 - 21 umgeben wird) ohne bzw. mit so geringer Formstabilität ausgebildet, dass sich die Trokarhülse 1 durch ihr Eigengewicht zusammenfaltet. Dies ist in Fig. 13 dargestellt. Die Trokarhülse 1 ist auf einer Unterlage 28 mit horizontaler Auflagefläche abgelegt, wobei die Längserstreckung der Trokarhülse 1 horizontal ausgerichtet ist. Durch das Eigengewicht der Trokarhülse 1 kommt es zur Auflage des oben liegenden Wandabschnitts auf dem untenliegenden Wandabschnitt.

Die Trokarhülse 1 besteht vorzugsweise, zumindest in ihrem kollabierbaren Abschnitt, aus einem textilen Material, insbesondere in Form eines Gewebes oder einer Maschenware, z. B. eines Gewirks. Die das textile Material ausbildenden Fäden bestehen vorzugsweise aus Seide, Baumwolle, Polypropylen, Nylon oder Gemischen hiervon. Auch andere Kunststoffe können eingesetzt werden.

Mindestens eine zusätzliche, nicht textile Lage der Trokarhülse 1 kann vorgesehen sein.

In anderen Ausführungsformen kann die Trokarhülse 1 auch von einem durchgehenden (folienartigen) Material gebildet werden, beispielsweise in Form von Polyurethan, Silikon, Teflon, Nylon oder Mischungen hiervon. Auch andere Kunststoffe können eingesetzt werden.

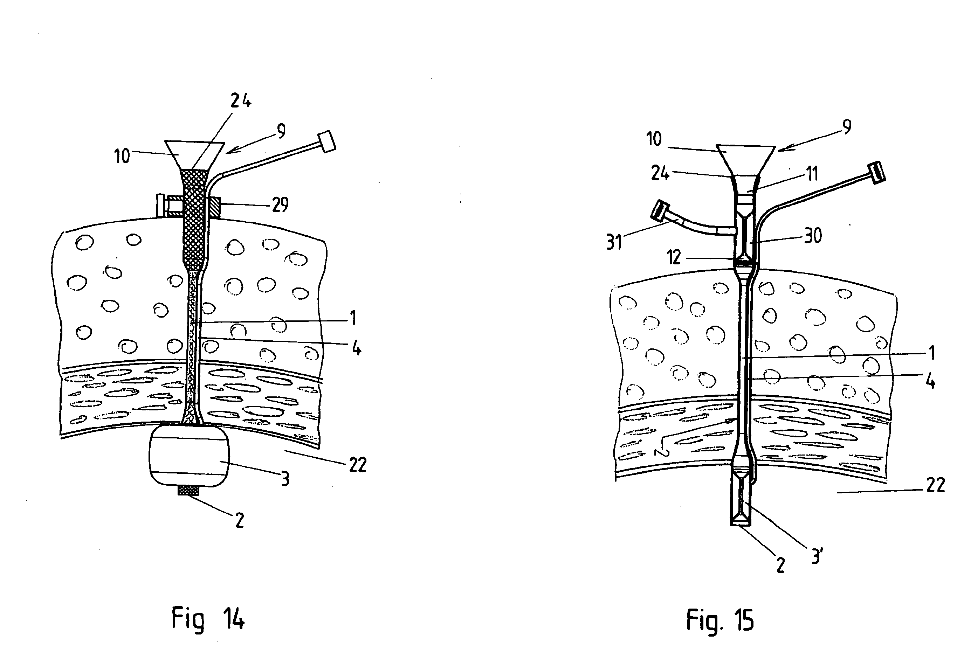

Eine weitere mögliche Ausführungsform ist in Fig. 14 dargestellt. Hier ist anstelle des proximalen Ringballons 13 ein proximales Klemmteil 29 vorgesehen, welches auf die Trokarhülse 1 von außen (achsial) aufgeschoben oder (radial) aufgesetzt ist und mit der Trokarhülse 1 verklemmbar ist. Das proximale Klemmteil 29 stützt sich zur Verhinderung eines weiteren Einschiebens der Trokarhülse 1 in die Körperhöhle 22 gegen die Haut 17 ab. Im ungeklemmten Zustand ist das Klemmteil 29 in Längsrichtung der Trokarhülse 1 verschiebbar bzw. von der Trokarhülse 1 abnehmbar.

Der Einsteckabschnitt 12 des Anschlussstücks 9 erstreckt sich hier bis über den Bereich, auf dem das Klemmteil 29 angeordnet ist hinaus und stützt die Trokarhülse 1 im Bereich des Klemmteils 29 nach innen ab.

Auch im zuvor anhand der Fig. 1 bis 11 beschriebenen Ausführungsbeispiele könnte sich der Einsteckabschnitt 12 über den Bereich hinaus erstrecken, in welchem der proximale Ringballon 13 auf der Trokarhülse 1 angeordnet ist. Der Einsteckabschnitt 12 würde dann die Trokarhülse 1 gegen den Druck des aufgeblasenen proximalen Ringballons 13 abstützen, sodass die Durchführung eines endoskopischen Instruments erleichtert wird.

Fig. 15 zeigt eine weitere mögliche Ausführungsform. Der distale Ringballon 3' liegt hier innerhalb eines distalen Endabschnitts der Trokarhülse 1 und erweitert den Außendurchmesser dieses distalen Endabschnitts der Trokarhülse 1 bei seiner Befüllung. Beim Einführen des endoskopischen Instruments 25 wird dieses durch den distalen Ringballon 3' hindurchgeschoben, wobei dieser distale Ringballon 3' eine zusätzliche Abdichtung gegen den Austritt von Gas aus der Körperhöhle 22 bilden kann.

Weiters ist das Anschlussstück 9 in dieser Ausführungsform mit einer zusätzlichen inneren Dichtung versehen, die als im Inneren des Einsteckabschnitts 12 angeordneter Ringballon 30 ausgebildet ist, der über ein Befüllröhrchen 31 befüllbar ist. In anderer Weise ausgebildete innere Dichtungen des Anschlussstücks 9 können vorgesehen sein.

Ein proximaler Ringballon 13 oder proximales Klemmteil 29 sind in Fig. 15 nicht eingezeichnet, können aber vorzugsweise ebenfalls eingesetzt werden.

Der in Fig. 15 dargestellte distale Ringballon könnte auch im anhand der Fig. 1 bis 11 beschriebenen Ausführungsbeispiel anstelle des dort gezeigten distalen Ringballons 3 eingesetzt werden.

Im anhand der Fig. 1 bis 11 beschriebenen Ausführungsbeispiel könnte auch ein Anschlussstück 9 mit einer zusätzlichen inneren Dichtung eingesetzt werden.

**Legende**

| zu den Hinweisziffern: | | | |
|---|---|---|---|
| 1 | Trokarhülse | 16 | Spritze |
| 2 | distales Ende | 17 | Haut |
| 3, 3' | distaler Ringballon | 18 | Gewebeschicht |
| 3a | Basisabschnitt | 19 | Gewebeschicht |
| 3b | Membran | 20 | Gewebeschicht |
| 4 | Befüllröhrchen | 21 | Gewebeschicht |
| 5 | Anschluss | 22 | Körperhöhle |
| 6 | Spritze | 23 | Schere |
| 7 | Dorn | 24 | proximales Ende |
| 8 | Spitze | 25 | endoskopisches Instrument |
| 9 | Anschlussstück | 26 | Abstützteil |
| 10 | Zuführabschnitt | 27 | Zustellteil |
| 11 | Durchgangskanal | 28 | Unterlage |
| 12 | Einsteckabschnitt | 29 | proximales Klemmteil |
| 13 | proximaler Ringballon | 30 | Ringballon |
| 14 | Befüllröhrchen | 31 | Befüllröhrchen |
| 15 | Anschluss | 32 | Verbindungsabschnitt |

## Patentansprüche

1. Medizinische Vorrichtung zum Einführen von endoskopischen Instrumenten (25) in eine Körperhöhle (22) eines Patienten bei einer endoskopischen Operation, mit einer Trokarhülse (1), welche ein distales Ende (2) zur Anordnung in der Körperhöhle (22) aufweist, und mit einem einen Durchgangskanal (11) aufweisenden Anschlussstück (9), welches im Einsatzzustand der Vorrichtung an einem proximalen Ende (24) der Trokarhülse (1) mit der Trokarhülse (1) verbunden ist, wobei das Anschlussstück (9) einen Zuführabschnitt (10), über welchen sich der Durchgangskanal zum von der Trokarhülse (1) abgelegenen Ende des Anschlussstücks (9) hin trichterförmig erweitert, und einen Einsteckabschnitt (12) aufweist, welcher in das durch Ablängen der Trokarhülse (1) an einer beliebigen Stelle zumindest innerhalb eines Verbindungsabschnitts (32) der Längserstreckung der Trokarhülse (1) ausbildbare proximale Ende (24) der Trokarhülse (1) einsteckbar ist, **dadurch gekennzeichnet, dass** die Trokarhülse (1) zumindest einen Abschnitt ihrer Längserstreckung aufweist, über welchen sie derart kollabierbar ausgebildet ist, **dass** sich die Trokarhülse (1) im auf einer Unterlage (28) mit horizontaler Auflagefläche abgelegten Zustand, wobei die Längserstreckung der Trokarhülse (1) horizontal liegt, zumindest über den Abschnitt Ihrer Längserstreckung, über welchen sie kollabierbar ausgebildet ist, zusammenfaltet, wobei bezogen auf den Querschnitt gegenüberliegende Wandabschnitte der Trokarhülse (1) aufeinander aufliegen, und dass zur Verankerung der Trokarhülse (1) gegen ein Herausziehen aus der Körperhöhle (22) während der Operation die Vorrichtung einen fest mit der Trokarhülse (1) verbundenen, mit einem Füllmedium befüllbaren distalen Ringballon (3, 3') aufweist.

2. Vorrichtung nach Anspruch 1, **dadurch gekennzeichnet, dass** die Trokarhülse (1) ein textiles Material aufweist, vorzugsweise vom textilen Material gebildet wird.

3. Vorrichtung nach Anspruch 1 oder 2, **dadurch gekennzeichnet, dass** zumindest im Abschnitt der Längserstreckung der Trokarhülse (1), über welchen sie kollabierbar ausgebildet ist, bezogen auf den Querschnitt der Trokarhülse (1) gegenüberliegende Wandabschnitte der Trokarhülse (1) durch eine Kraft bis auf Anlage zusammendrückbar sind, welche weniger als 6N beträgt, vorzugsweise weniger als 3N.

4. Vorrichtung nach einem der Ansprüche 1 bis 3, **dadurch gekennzeichnet, dass** der distale Ringballon (3) die Trokarhülse (1) umgibt, wobei sich der Außendurchmesser des distalen Ringballons (3) beim Befüllen vergrößert.

5. Vorrichtung nach Anspruch 4, **dadurch gekennzeichnet, dass** der distale Ringballon (3') innerhalb der Trokarhülse liegt und den Außendurchmesser der Trokarhülse (1) bei seiner Befüllung vergrößert.

6. Vorrichtung nach einem der Ansprüche 1 bis 5, **dadurch gekennzeichnet, dass** ein Befüllröhrchen (4) zum Befüllen des distalen Ringballons (3, 3') mit dem Füllmedium an der Außenseite der Trokarhülse in Richtung von deren Längserstreckung läuft.

7. Vorrichtung nach einem der Ansprüche 1 bis 6, **dadurch gekennzeichnet, dass** zur Verankerung der Trokarhülse (1) gegen ein weiteres Hineinschieben in die Körperhöhle (22) hinein die Vorrichtung ein Verankerungsteil (13, 29) aufweist, welches in einem geöffneten Zustand gegenüber der Trokarhülse (1) in deren Längsrichtung verschiebbar ist und/oder an verschiedenen Stellen der Längserstreckung der Trokarhülse (1) an dieser anlegbar ist und welches in einem Klemmzustand gegen eine Verschiebung gegenüber der Trokarhülse (1) gesichert ist.

8. Vorrichtung nach Anspruch 7, **dadurch gekennzeichnet, dass** das Verankerungsteil von einem proximalen Ringballon (13) gebildet wird, der die Trokarhülse (1) umgibt und dessen Innendurchmesser sich beim Befüllen verringert.

9. Vorrichtung nach Anspruch 8, **dadurch gekennzeichnet, dass** das Verankerungsteil von einem proximalen Klemmteil (29) gebildet wird, welches gegen die Trokarhülse (1) verklemmbar ist.

10. Vorrichtung nach einem der Ansprüche 1 bis 9, **dadurch gekennzeichnet, dass** die Trokarhülse (1) zumindest im Verbindungsabschnitt (32) elastisch dehnbar ausgebildet ist.

11. Vorrichtung nach einem der Ansprüche 1 bis 10, **dadurch gekennzeichnet, dass** die Trokarhülse (1) im bezogen auf ihren Querschnitt zu einem Kreisring geöffneten, aber ungedehnten Zustand zumindest über den Verbindungsabschnitt (32) einen konstanten Innendurchmesser aufweist.

## Claims

1. Medical device for inserting endoscopic instruments (25) into a body cavity (22) of a patient in an endoscopic operation, having a trocar sleeve (1) which has a distal end (2) for arrangement in the body cavity (22), and having a connecting piece (9) which has a through-passage (11) and which, when the device is in the inserted state, is connected to the trocar sleeve (1) at a proximal end (24) of said trocar sleeve (1), the connecting piece (9) having an feed-in portion (10) over which the through-passage enlarges in a funnel shape to the end of the connecting piece (9) remote from the trocar sleeve (1) and having an insertion portion (12) which can be inserted in the proximal end (24) of the trocar sleeve (1), which proximal end (24) can be created by cutting the trocar sleeve (1) to length at any desired point at least within a connecting portion (32) of the longitudinal extent of the trocar sleeve (1), **characterised in that** the trocar sleeve (1) has at least a portion of its longitudinal extent over which it is adapted to be collapsible in such a way that the trocar sleeve (1), in the state in which it is laid down on a support (28) having a horizontal supporting surface, in which case the longitudinal extent of the trocar sleeve (1) lies horizontally, collapses at least over that portion of its longitudinal extent over which it is adapted to be collapsible, in which case portions of the wall of the trocar sleeve (1) which are opposite one another across the cross-section rest against one another, and **in that**, to anchor the trocar sleeve (1) against being withdrawn from the body cavity (22) during the operation, the device has a distal annular balloon (3, 3') which is solidly connected to the trocar sleeve (1) and which can be filled with a filling medium.

2. Device according to claim 1, **characterised in that** the trocar sleeve (1) has a textile material and is preferably formed from textile material.

3. Device according to claim 1 or 2, **characterised in that**, at least **in that** portion of the longitudinal extent of the trocar sleeve (1) over which it is adapted to be collapsible, portions of the wall of the trocar sleeve (1) which are opposite one another across the cross-section of the trocar sleeve (1) can be pressed together until in contact by a force which is less than 6N and preferably less than 3N.

4. Device according to one of claims 1 to 3, **characterised in that** the distal annular balloon (3) surrounds the trocar sleeve (1), the outside diameter of the distal annular balloon (3) increasing as it is filled.

5. Device according to claim 4, **characterised in that** the distal annular balloon (3') is situated inside the trocar sleeve (1) and increases the outside diameter of the trocar sleeve (1) as said distal annular balloon (3') is filled.

6. Device according to one of claims 1 to 5, **characterised in that** a filling tube (4) for filling the distal annular balloon (3, 3') with the filling medium runs along the outside of the trocar sleeve in the direction defined by the latter's longitudinal extent.

7. Device according to one of claims 1 to 6, **characterised in that**, to anchor the trocar sleeve (1) against being urged further into the body cavity (22), the device has an anchoring part (13, 29) which, in an opened state, is displaceable relative to the trocar sleeve (1) in the latter's longitudinal direction and/or can be placed against the trocar sleeve (1) at various points along the latter's longitudinal extent, and which, in a clamped state, is secured against displacement relative to the trocar sleeve (1).

8. Device according to claim 7, **characterised in that** the anchoring part is formed by a proximal annular balloon (13) which surrounds the trocar sleeve (1) and reduces the latter's inside diameter as it is filled.

9. Device according to claim 8, **characterised in that** the anchoring part is formed by a proximal clamping part (29) which can be clamped against the trocar sleeve (1) .

10. Device according to one of claims 1 to 9, **characterised in that** the trocar sleeve (1) is adapted to be elastically expansible at least in the connecting portion (32).

11. Device according to one of claims 1 to 10, **characterised in that**, in the state where it is opened into an annular ring across the cross-section but is unexpanded, the trocar sleeve (1) is of a constant inside diameter at least over the connecting portion (32).

## Revendications

1. Dispositif médical pour permettre l'introduction d'instruments endoscopiques (25) dans une cavité corporelle (22) d'un patient lors d'une opération par endoscopie, comprenant une douille de trocart (1) qui comporte une extrémité distale (2) destinée à être positionnée dans la cavité corporelle (22), ainsi qu'une pièce de connexion (9) comprenant un canal traversant (11) et qui, lorsque le dispositif est utilisé est reliée à la douille de trocart (1) à une extrémité proximale (24) de cette douille de trocart (1), la pièce de connexion (9) comprenant un segment d'introduction (10) au niveau duquel le canal de passage s'élargi en forme d'entonnoir vers l'extrémité de la pièce de connexion (9) isolée de la douille de trocart (1), et un segment d'enfichage (12) qui peut être inséré dans l'extrémité proximale (24) de la douille de trocart (1) pouvant être formée par découpe de la douille de trocart (1) à un endroit quelconque au moins à la partie interne d'un segment de liaison (32) de l'étendue longitudinale de cette douille de trocart (1),
**caractérisé en ce que**
la douille de trocart (1) comporte au moins un segment de son extension longitudinale au niveau duquel elle est réalisée de manière à pouvoir s'affaisser de sorte que cette douille de trocart (1) se replie lorsqu'elle est déposée sur un support (28) ayant une surface d'appui horizontale, l'extension longitudinale de la douille de trocart étant horizontale, au moins au niveau de la partie de son extension longitudinale sur laquelle elle est réalisée de manière à pouvoir s'affaisser, les segments de parois opposés par rapport à la section de la douille de trocart (1) s'appuyant les uns sur les autres, et, pour permettre de bloquer la douille de trocart (1) pour empêcher son extraction de la cavité corporelle (22) pendant l'opération, le dispositif comporte un ballon annulaire distal (3,3') pouvant être rempli avec un fluide de remplissage, relié fermement à la douille de trocart (1).

2. Dispositif conforme à la revendication 1,
**caractérisé en ce que**
la douille de trocart (1) comporte un matériau textile et est de préférence réalisée en un matériau textile.

3. Dispositif conforme à la revendication 1 ou 2,
**caractérisé en ce qu'**
au moins sur la partie de l'étendue longitudinale de la douille de trocart (1) au niveau de laquelle celle-ci est réalisée de façon à pouvoir s'affaisser, les segments de paroi de la douille de trocart (1) opposés par rapport à la section de la douille de trocart (1) peuvent être comprimés l'un contre l'autre jusqu'à venir en appui par une force inférieure à 6 N et de préférence inférieure à 3 N.

4. Dispositif conforme à l'une des revendications 1 à 3,
**caractérisé en ce que**
le ballon annulaire distal (3) entoure la douille de trocart (1), le diamètre externe du ballon annulaire distal (3) augmentant lors du remplissage.

5. Dispositif conforme à la revendication 4,
**caractérisé en ce que**
le ballon annulaire distal (3') est situé à la partie interne de la douille de trocart et augmente le diamètre externe de la douille de trocart (1) lors de son remplissage.

6. Dispositif conforme à l'une des revendications 1 à 5,
**caractérisé en ce qu'**
un petit tube de remplissage (4) permettant de remplir le ballon annulaire distal (3, 3') avec le fluide de remplissage s'étend sur la face externe de la douille de trocart en direction de son étendue longitudinale.

7. Dispositif conforme à l'une des revendications 1 à 6,
**caractérisé en ce que**
pour permettre de bloquer la douille de trocart (1) pour empêcher une poursuite de l'insertion de celle-ci dans la cavité corporelle (22), le dispositif comporte une pièce de blocage (13, 29) qui peut, dans un état ouvert coulisser vis-à-vis de la douille de trocart (1), sur son étendue longitudinale, et/ou peut être positionnée sur la douille de trocart (1) à différents endroits de son étendue longitudinale, et qui dans un état de serrage est bloquée pour empêcher un coulissement par rapport à la douille de trocart (1).

8. Dispositif conforme à la revendication 7,
**caractérisé en ce que**
la pièce de blocage est formée par un ballon annulaire proximal (13) qui entoure la douille de trocart (1) et dont le diamètre interne diminue lors du remplissage.

9. Dispositif conforme à la revendication 8,
**caractérisé en ce que**
la pièce de blocage est formée par une pièce de serrage proximale (29) qui peut être bloquée par serrage contre la douille de trocart (1).

10. Dispositif conforme à l'une des revendications 1 à 9,
**caractérisé en ce que**
la douille de trocart (1) est étirable élastiquement au moins dans le segment de liaison (32).

11. Dispositif conforme à l'une des revendications 1 à 10,
**caractérisé en ce que**
la douille de trocart (1) a un diamètre interne constant dans son état ouvert en un anneau circulaire selon sa section mais non étiré, au moins au niveau du segment de liaison (32).
